(19) **Europäisches Patentamt / European Patent Office / Office européen des brevets**

(11) **EP 3 563 759 A1**

(12) # EUROPEAN PATENT APPLICATION

(43) Date of publication:
06.11.2019 Bulletin 2019/45

(51) Int Cl.:
*A61B 5/021* (2006.01)    *A61B 5/0295* (2006.01)
*A61B 5/00* (2006.01)    *A61B 5/024* (2006.01)

(21) Application number: 18170248.1

(22) Date of filing: 01.05.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
- **MUEHLSTEFF, Jens**
  **5656 AE Eindhoven (NL)**
- **ATALLAH, Louis Nicolas**
  **5656 AE Eindhoven (NL)**
- **SERTEYN, Aline Anne Marie**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **APPARATUS FOR DETERMINING A STRESS AND/OR PAIN LEVEL**

(57)    An apparatus (100) for determining a stress and/or pain level of a subject comprises a processor (102). The processor (102) is configured to acquire, from a photoplethysmography sensor (104), a photoplethysmography signal obtained from the subject. The processor (102) is also configured to identify a characteristic of the acquired photoplethysmography signal. The characteristic is normalized for blood pressure. The processor (102) is further configured to determine the stress and/or pain level of the subject from the acquired photoplethysmography signal based on the normalized characteristic.

Fig. 4A (Prior Art)

$$R = \frac{PPG}{BP}$$

Fig. 4B

**Description**

FIELD OF THE INVENTION

The disclosure relates to an apparatus and method for determining a stress and/or pain level of a subject.

BACKGROUND OF THE INVENTION

Currently, pain and stress monitoring for awake patients is visualized on an analogue scale, which allows the patient themselves to indicate their pain and/or stress level. However, the required attention of the patient towards feeling pain and/or stress can bias this estimation and the assessment can thus be unreliable. The reliability of pain and stress monitoring can be increased by using objective measures to reflect pain and/or stress levels, which do not require the attention of the patient. For example, objective measures may involve estimating a body response to nociception. These objective measures are even more important for sedated patients, particularly where it is necessary to indicate to clinicians how to adequately administer analgesic medication. There exist several techniques for quantifying pain and/or stress, which are based on the manner in which vital signs react to nociception.

An example of one such a technique uses electrocardiogram (ECG) and photoplethysmography (PPG) signals to determine a pulse transit time (PTT) from heart to hand, which is taken to be indicative of stress and/or pain. The aim of this technique is to monitor for decreases in PTT, which are interpreted as stress and/or pain, and then administer analgesics to cause the PTT to increase. In another example of a technique for pain monitoring, features are extracted from PPG and galvanic skin response (GSR) signals and transformed into a single measure that is used to assess pain. Another example technique for pain monitoring involves performing a time-frequency analysis on a heart rate variability signal from an ECG using wavelet transforms. The output of this analysis is used to observe respiratory sinus arrhythmia changes due to pain. Other techniques that are often used during anesthesia use blood pressure (BP) as well as blood pressure changes (e.g. measured invasively by arterial-lines or non-invasively by cuff-based blood pressure measurement devices) as pain indicators for conscious patients.

Another technique that is widely used as a pain and/or stress indicator due to its simplicity and ease of use involves using an amplitude of a photoplethysmography (PPG) signal obtained from a peripheral part of the body of a patient as an indicator of pain and/or stress in the patient. In this technique, the amplitude of the photoplethysmography signal is expected to decrease during a painful and/or stressful stimuli (e.g. an incision), whereas an increase in the photoplethysmography signal is expected when the patient is more relaxed. The amplitude of the photoplethysmography signal is expected to decrease during a painful and/or stressful stimuli since such a stimuli causes the autonomous nervous system to trigger smooth muscle around vessels to constrict in order to centralize essential body functions. This is known as vasoconstriction. However, while this technique has proven to be popular for indicating painful and/or stressful events, it still lacks accuracy and reliability.

In particular, painful and/or stressful stimuli are not only characterized by vasoconstriction but they are also associated with an increase in blood pressure. Thus, the blood pressure response of a patient can also influence the amplitude of a photoplethysmography signal obtained from a peripheral part of the body of the patient. While a painful and/or stressful stimuli causes a decrease in the amplitude of a photoplethysmography signal obtained from a peripheral part of the body of a patient due to vasoconstriction, an increase in blood pressure (e.g. systolic blood pressure (SBP), pulse pressure (PP), mean blood pressure (MBP) or diastolic blood pressure (DBP)) can result in an increase in the amplitude of the photoplethysmography signal obtained from the peripheral part of the body of the patient. As mentioned earlier, an increase in the amplitude of a photoplethysmography signal is identified as a more relaxed patient. Thus, as a consequence, it is possible for a patient to be identified as relaxed despite the fact that the patient may in fact be in more pain and/or stressed.

As such, an amplitude of a peripherally measured photoplethysmography signal according to existing techniques as an indicator for painful and/or stressful events currently lacks specificity, which can inevitably lead to wrong conclusions being made on the treatment that is appropriate for a patient when a blood pressure effect dominates the photoplethysmography signal.

SUMMARY OF THE INVENTION

As noted above, a limitation with existing techniques is that they can result in incorrect indications of a pain and/or stress level for a subject. As such, existing techniques can be inaccurate and unreliable. It would thus be valuable to have an improved manner in which to determine a stress and/or pain level of a subject, which aims to address the existing problems.

Therefore, according to a first aspect, there is provided an apparatus for determining a stress and/or pain level of a subject. The apparatus comprises a processor configured to acquire, from a photoplethysmography sensor, a photoplethysmography signal obtained from the subject and identify a characteristic of the acquired photoplethysmography signal. The characteristic is normalized for blood pressure. The processor is also configured to determine the stress and/or pain level of the subject from the acquired photoplethysmography signal based on the normalized characteristic.

In some embodiments, the characteristic may be normalized for blood pressure by the processor being configured to acquire, from the photoplethysmography sensor, a photoplethysmography signal obtained from a central part of the body of the subject.

In some embodiments, the characteristic may be normalized for blood pressure by the processor being configured to acquire, from the photoplethysmography sensor, a photoplethysmography signal obtained from a peripheral part of the body of the subject, acquire, from a blood pressure sensor, a blood pressure measurement for the subject, and normalize the characteristic with the blood pressure measurement for the subject to obtain the characteristic normalized for blood pressure.

In some embodiments, the processor may be configured to normalize the characteristic with the blood pressure measurement for the subject by determining a ratio of the characteristic to the blood pressure measurement for the subject to obtain the characteristic normalized for blood pressure.

In some embodiments, the blood pressure measurement may comprise a systolic blood pressure measurement, a diastolic blood pressure measurement, a mean blood pressure measurement, or a pulse pressure measurement.

In some embodiments, the characteristic may be normalized for blood pressure by the processor being configured to acquire, from a first photoplethysmography sensor, a first photoplethysmography signal obtained from a first part of the body of the subject, acquire, from a second photoplethysmography sensor, a second photoplethysmography signal obtained from a second part of the body of the subject, identify a first characteristic of the first photoplethysmography signal and a corresponding second characteristic of the second photoplethysmography signal, and normalize the first characteristic with the corresponding second characteristic to obtain the characteristic normalized for blood pressure.

In some embodiments, the first part of the body of the subject may be different to the second part of the body of the subject.

In some embodiments, one of the first part of the body of the subject and the second part of the body of the subject may be a peripheral part of the body of the subject and the other of the first part of the body of the subject and the second part of the body of the subject may be a central part of the body of the subject.

In some embodiments, the processor may be configured to normalize the first characteristic with the second characteristic by determining a ratio of the first characteristic to the second characteristic to obtain the characteristic normalized for blood pressure.

In some embodiments, the processor may be configured to normalize the characteristic for blood pressure by determining a pulse transit time for the subject and normalizing the characteristic with the determined pulse transit time for the subject to obtain the characteristic normalized for blood pressure.

In some embodiments, the processor may be configured to normalize the characteristic with the determined pulse transit time for the subject by determining a ratio of the characteristic to the determined pulse transit time for the subject to obtain the characteristic normalized for blood pressure.

In some embodiments, the processor may be configured to determine the stress and/or pain level of the subject by identifying whether one or more areas of the acquired photoplethysmography signal are indicative of an increase in the stress and/or pain level of the subject based on the normalized characteristic.

In some embodiments, the characteristic of the acquired photoplethysmography signal may comprise an amplitude of the acquired photoplethysmography signal.

According to a second aspect, there is provided a method for determining a stress and/or pain level of a subject. The method comprises acquiring, from a photoplethysmography sensor, a photoplethysmography signal obtained from the subject and identifying a characteristic of the acquired photoplethysmography signal. The characteristic is normalized for blood pressure. The method also comprises determining the stress and/or pain level of the subject from the acquired photoplethysmography signal based on the normalized characteristic.

According to a third aspect, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing techniques are addressed. In particular, according to the above-described aspects and embodiments, a characteristic of the acquired photoplethysmography signal on which the stress and/or pain level determination is based is normalized for blood pressure. In this way, the effect of blood pressure (such as pulse pressure, mean blood pressure, etc.) on the acquired photoplethysmography signal is reduced to more accurately infer a pain and/or stress response of the subject. The specificity of the characteristic of the acquired photoplethysmography signal on which the stress and/or pain level determination is based is increased for the inference of the pain and/or stress status of the subject. This increase in specificity means that the pain and/or stress status of the subject can be discriminated from any blood pressure changes of the subject.

Thus, the above-described aspects and embodiments provide more accurate and more reliable determinations of the stress and/or pain level of a subject. In this way, false interpretation of the stress and/or pain status of a subject is reduced such that more appropriate treatment and care can be provided to the subject. There is thus provided an improved manner in which to determine a stress and/or pain level of a subject, which overcomes existing problems.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a block diagram of an apparatus according to an embodiment;
Fig. 2 is flow chart illustrating a method according to an embodiment;
Figs. 3A-B are illustrations of signals acquired using an existing technique and signals acquired using an apparatus according to an embodiment described herein;
Figs. 4A-B are illustrations of signals acquired using an existing technique and signals acquired using an apparatus according to another embodiment described herein;
Figs. 5A-B are illustrations of signals acquired using an existing technique and signals acquired using an apparatus according to another embodiment described herein;
Fig. 6 is an illustration of signals acquired using an apparatus according to some embodiments described herein; and
Fig. 7 is an illustration of an example output of signals acquired using an apparatus according to some embodiments described herein.

DETAILED DESCRIPTION OF EMBODIMENTS

As noted above, there is provided herein an improved manner in which to determine a stress and/or pain level of a subject (e.g. a patient or any other subject), which overcomes existing problems. Herein, the stress level of the subject can comprise a mental and/or physiological stress level of the subject. In cases of extreme physiological stress, the subject may enter into shock.

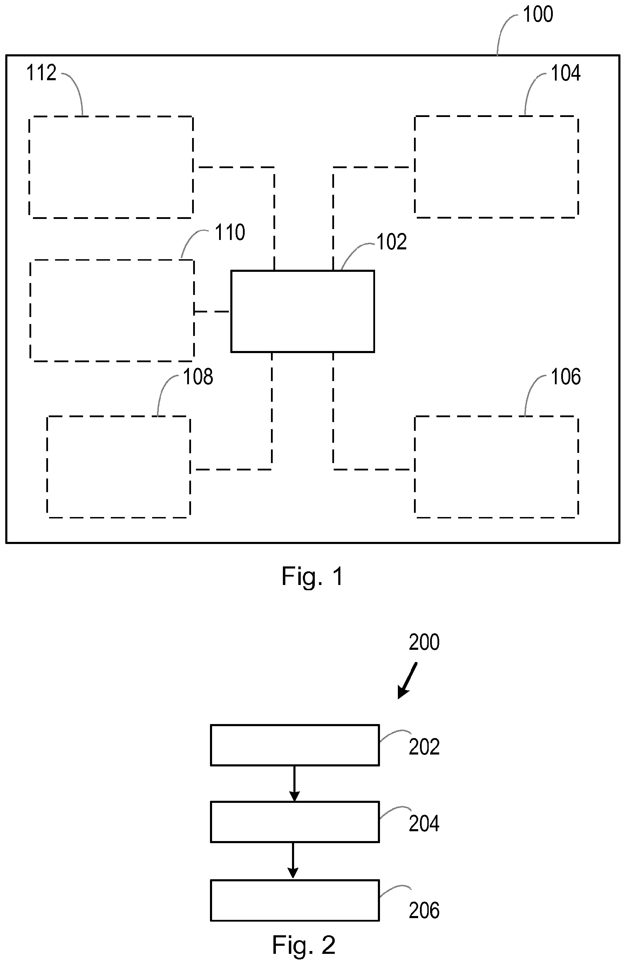

Fig. 1 illustrates an apparatus 100 for determining a stress and/or pain level of a subject. As illustrated in Fig. 1, the apparatus 100 comprises a processor 102. Briefly, the processor 102 of the apparatus 100 is configured to acquire, from a photoplethysmography (PPG) sensor 104, a photoplethysmography (PPG) signal obtained from the subject. The processor 102 of the apparatus 100 is also configured to identify a characteristic (or feature) of the acquired photoplethysmography signal. The characteristic is normalized for blood pressure. The processor 102 of the apparatus 100 is further configured to determine the stress and/or pain level of the subject from the acquired photoplethysmography signal based on the normalized characteristic.

Herein, the photoplethysmography signal may also be referred to as a photoplethysmogram. The photoplethysmography signal (or photoplethysmogram) referred to herein is defined as a measurement of changes in blood volume in the skin of the subject over time, where the measurements are obtained optically from the skin. For example, light can be used to illuminate the skin of the subject and changes in the amount of light reflected from the skin can be measured. The changes in the amount of light reflected from the skin of the subject are indicative of the changes in the blood volume.

The apparatus 100 may comprise one or more processors 102. The one or more processors 102 can be implemented in numerous ways, with software and/or hardware, to perform the various functions described herein. In some embodiments, each of the one or more processors 102 can be configured to perform individual or multiple steps of the method described herein. In particular implementations, the one or more processors 102 can comprise a plurality of software and/or hardware modules, each configured to perform, or that are for performing, individual or multiple steps of the method described herein. The one or more processors 102 may comprise one or more microprocessors, one or more multi-core processors and/or one or more digital signal processors (DSPs), one or more processing units, and/or one or more controllers (such as one or more microcontrollers) that may be configured or programmed (e.g. using software or computer program code) to perform the various functions described herein. The one or more processors 102 may be implemented as a combination of dedicated hardware (e.g. amplifiers, pre-amplifiers, analog-to-digital convertors (ADCs) and/or digital-to-analog convertors (DACs)) to perform some functions and one or more processors (e.g. one or more programmed microprocessors, DSPs and associated circuitry) to perform other functions.

As illustrated in Fig. 1, in some embodiments, the apparatus 100 may comprise the photoplethysmography sensor 104. However, it will be understood that, in other embodiments, the photoplethysmography sensor 104 may be external to (i.e. separate to or remote from) the apparatus 100. For example, in some embodiments, the photoplethysmography sensor 104 can be a separate entity or part of another apparatus (e.g. a device). The photoplethysmography sensor 104 is configured to obtain the photoplethysmography signal from the subject. The photoplethysmography sensor 104 can be any sensor, or any combination of sensors, suitable for obtaining a photoplethysmography signal (or a photoplethysmogram) from a subject. The photoplethysmography sensor 104 can be any type of optical sensor.

Examples of a photoplethysmography sensor 104 include, but are not limited to, a camera, one or a plurality of (e.g. an array of) photodetectors, a pulse oximeter, or any other sensor, or any combination of sensors, suitable for obtaining a photoplethysmography signal (or a photoplethysmogram) from a subject. The photoplethysmography sensor 104 can comprise a contact photoplethysmography sensor and/or a non-contact photoplethysmography sensor. A non-contact photoplethysmography sensor can be any photoplethysmography sensor that is configured to be remote from (or at a distance from) the subject 104, such that the photoplethysmography sensor 104 has no physical contact with the body of the subject. A contact photoplethysmography sensor 104 can be any photoplethysmography sensor that is configured to be in physical contact with the body of the subject. For example, the photoplethysmography sensor 104 may be configured to be worn (e.g. against the skin) on a part of the body of the subject (e.g. on a finger, a wrist and/or any other part of the body of the subject). In some of these embodiments, for example, a band configured to be worn around a part of the body of the subject may comprise the photoplethysmography sensor 104 or the photoplethysmography sensor 104 may be placed under a band configured to be worn around a part of the body of the subject.

In some embodiments, the photoplethysmography sensor 104 can be configured to obtain the photoplethysmography signal from a central part (or central site) of the body of the subject. A central part (or central site) of the body of the subject can be any location of the body of the subject that is close to (e.g. less than or equal to a predetermined distance from) a vital organ of the body of the subject. Examples of a vital organ of the body of the subject include, but are not limited to, the heart of the subject, a lung of the subject, the brain of the subject, or any other vital organ of the subject. Examples of a central part of the body of the subject may include, but are not limited to, the forehead of the subject, the nose of the subject (e.g. an ala of the nose of the subject or the septum of the nose of the subject), a part of the body behind an ear of the subject, the concha of an ear of the subject, the neck of the subject, the upper chest area of the subject, or any other central part of the body of the subject. Thus, for example, a central part of the body of the subject according to some embodiments can be any location of the body of the subject that is less than or equal to 10 cm (for example less than or equal to 9 cm, for example less than or equal to 8 cm, for example less than or equal to 7 cm, for example less than or equal to 6 cm, for example less than or equal to 5 cm, for example less than or equal to 4 cm, for example less than or equal to 3 cm, for example less than or equal to 2 cm, for example less than or equal to 1 cm, or less than or equal to any other integer or non-integer distance that is less than 10 cm) from a vital organ of the body of the subject.

In other embodiments, the photoplethysmography sensor 104 can be configured to obtain the photoplethysmography signal from a peripheral part (or peripheral site) of the body of the subject. A peripheral part of the body of the subject can be any location of the body of the subject that is far away from (e.g. greater than the predetermined distance from) a vital organ of the body of the subject, such as any of those mentioned earlier. Examples of a peripheral part (or peripheral site) of the body of the subject may include, but are not limited to, a finger of the subject, an ear lobe of the subject, a foot of the subject, or any other peripheral part of the body of the subject. Thus, for example, a peripheral part of the body of the subject according to some embodiments can be any location of the body of the subject that is greater than 1 cm (for example greater than 2 cm, for example greater than 3 cm, for example greater than 4 cm, for example greater than 5 cm, for example greater than 6 cm, for example greater than 7 cm, for example greater than 8 cm, for example greater than 9 cm, for example greater than 10 cm, or greater than any other integer or non-integer distance that is greater than 1 cm) from a vital organ of the body of the subject.

As illustrated in Fig. 1, in some embodiments, the apparatus 100 may comprise a communications interface (or communications circuitry) 106. Alternatively or in addition, the communications interface 106 may be external to (e.g. separate to or remote from) the apparatus 100. The communications interface 106 can be for enabling the apparatus 100, or components of the apparatus 100, to communicate with and/or connect to one or more other components, sensors, interfaces, devices, or memories (such as any of those described herein). For example, the communications interface 106 can be for enabling the processor of the apparatus 100 to communicate with and/or connect to the photoplethysmography sensor 104 described earlier. The communications interface 106 may enable the apparatus 100, or components of the apparatus 100, to communicate and/or connect in any suitable way. For example, the communications interface 106 may enable the apparatus 100, or components of the apparatus 100, to communicate and/or connect wirelessly, via a wired connection, or via any other communication (or data transfer) mechanism. In some wireless embodiments, for example, the communications interface 106 may enable the apparatus 100, or components of the apparatus 100, to use radio frequency (RF), Bluetooth, or any other wireless communication technology to communicate and/or connect.

As illustrated in Fig. 1, in some embodiments, the apparatus 100 may comprise a memory 108. Alternatively or in addition, the memory 108 may be external to (e.g. separate to or remote from) the apparatus 100. The processor of the apparatus 100 may be configured to communicate with and/or connect to the memory 108. The memory 108 may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, the memory 108 can be configured to store program code that can be executed by the processor 102 to cause the apparatus 100 to operate in the manner described herein.

Alternatively or in addition, in some embodiments, the memory 108 can be configured to store information required by or resulting from the method described herein. For example, in some embodiments, the memory 108 may be configured to store any one or more of the acquired photoplethysmography signal, the identified characteristic of the acquired photoplethysmography signal, the characteristic normalized for blood pressure, the determined stress and/or pain level of the subject, or any other information, or any combination of information, required by or resulting from the method described herein. In some embodiments, the processor 102 of the apparatus 100 can be configured to control the memory 108 to store information required by or resulting from the method described herein.

As illustrated in Fig. 1, in some embodiments, the apparatus 100 may comprise a user interface 110. Alternatively or in addition, the user interface 110 may be external to (e.g. separate to or remote from) the apparatus 100. The processor 102 of the apparatus 100 may be configured to communicate with and/or connect to a user interface 110. The user interface 110 can be configured to render (e.g. output, display, or provide) information required by or resulting from the method described herein. For example, in some embodiments, the user interface 110 may be configured to render (e.g. output, display, or provide) one or more of the acquired photoplethysmography signal, the identified characteristic of the acquired photoplethysmography signal, the characteristic normalized for blood pressure, the determined stress and/or pain level of the subject, or any other information, or any combination of information, required by or resulting from the method described herein. Alternatively or in addition, the user interface 110 can be configured to receive a user input. For example, the user interface 110 may allow a user to manually enter information or instructions, interact with and/or control the apparatus 100. Thus, the user interface 110 may be any user interface that enables the rendering (or outputting, displaying, or providing) of information and, alternatively or in addition, enables a user to provide a user input. In some embodiments, the processor 102 of the apparatus 100 can be configured to control the user interface 110 to operate in the manner described herein.

For example, the user interface 110 may comprise one or more switches, one or more buttons, a keypad, a keyboard, a mouse, a touch screen or an application (e.g. on a smart device such as a tablet, a smartphone, or any other smart device), a display or display screen, a graphical user interface (GUI) such as a touch screen, or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input.

As illustrated in Fig. 1, in some embodiments, the apparatus 100 may comprise a blood pressure sensor 112. Alternatively or in addition, the blood pressure sensor 112 may be external to (e.g. separate to or remote from) the apparatus 100. The processor 102 of the apparatus 100 may be configured to communicate with and/or connect to the blood pressure sensor 112. The blood pressure sensor 112 can be configured to obtain a blood pressure measurement for the subject. The blood pressure sensor 112 can thus be any type of sensor suitable for obtaining a blood pressure measurement for the subject.

A person skilled in the art will be aware of a variety of sensors that may be suitable for obtaining a blood pressure measurement for the subject. However, examples of the blood pressure sensor 112 may include, but are not limited to, cuff-based blood pressure sensors or cuff-less blood pressure sensors. A cuff-based blood pressure sensor involves a wearable cuff that is inflated around a part of the body (e.g. an arm, a wrist, a finger, etc.) of the subject. In these embodiments, a blood pressure measurement for the subject may be obtained during deflation of the wearable cuff. A cuff-less blood pressure sensor, for example, can comprise an optical biometric sensor configured to extract pulsatile blood flow from a part of the body of the subject to obtain a blood pressure measurement for the subject. For example, a pulse transit time or pulse arrival time may be used as blood pressure surrogates, which can involve the detection of a first pulse or electrical signal and a second pulse signal. In other examples, a blood pressure measurement may be acquired from a signal such as an electrocardiogram (ECG), an impedance cardiogram, a heart sound signal, or a radar signal. Thus, an obtained blood pressure measurement can be a blood pressure measurement that is either obtained directly from the body of the subject or a blood pressure measurement that is obtained indirectly from the body of the subject by estimation. Although examples have been provided for the manner in which the blood pressure measurement may be acquired, it will be understood that any other sensor, or any combination of sensors, suitable for obtaining (either directly or indirectly) a blood pressure measurement for the subject can be used.

Although not illustrated in Fig 1, the apparatus 100 may comprise a battery or other power supply for powering the apparatus 100 or means for connecting the apparatus 100 to a mains power supply. It will also be understood that the apparatus 100 may comprise any other component to those described herein or any combination of components.

Fig. 2 illustrates a method 200 of operating an apparatus 100 (as described earlier with reference to Fig. 1) for determining a stress and/or pain level of a subject according to an embodiment. As described earlier, the apparatus 100 comprises a processor 102. The method 200 illustrated in Fig. 2 can generally be performed by or under the control of the processor 102. With reference to Fig. 2, at block 202, a photoplethysmography (PPG) signal obtained from the subject is acquired from a photoplethysmography (PPG) sensor 104. More specifically, as mentioned earlier, the photoplethysmography (PPG) signal obtained from the subject is acquired by the processor 102 of the apparatus 100 from the photoplethysmography sensor 104.

At block 204 of Fig. 2, a characteristic (or feature) of the acquired photoplethysmography signal is identified. In some embodiments, the characteristic can comprise an amplitude of the acquired photoplethysmography signal (PPGA), an amplitude of the acquired photoplethysmography signal (PPGA) divided by a direct current (DC) component of the acquired photoplethysmography signal, an overall signal range of the acquired photoplethysmography signal, a signal power of the acquired photoplethysmography signal, a signal energy of the acquired photoplethysmography signal, or any other characteristic of the acquired photoplethysmography signal. The amplitude of the acquired photoplethysmography signal can, for example, comprise a root mean squared amplitude of the acquired photoplethysmography signal or a peak-to-peak amplitude of the acquired photoplethysmography signal. In some embodiments, the amplitude of the acquired photoplethysmography signal can be defined as the difference between a maximum of the acquired photoplethysmography signal and a minimum of the acquired photoplethysmography signal (e.g. within a heart period or otherwise defined physiological time period, such as a respiration phase), or as the energy of the acquired photoplethysmography signal in a certain frequency band.

In any of the embodiments described herein, the characteristic of the acquired photoplethysmography signal is normalized for blood pressure. The characteristic of the acquired photoplethysmography signal can be normalized for blood pressure in a variety of ways, some of which will be described later.

Returning back to Fig. 2, at block 206, the stress and/or pain level of the subject is determined from the acquired photoplethysmography signal based on the normalized characteristic of the acquired photoplethysmography signal. In some embodiments, the processor 102 can be configured to determine the stress and/or pain level of the subject by identifying whether one or more areas (or portions) of the acquired photoplethysmography signal are indicative of an increase in the stress and/or pain level of the subject based on the normalized characteristic.

For example, in embodiments where the characteristic is an amplitude of the acquired photoplethysmography signal, an area (or portion) of the acquired photoplethysmography signal comprising a decrease in the normalized amplitude of the acquired photoplethysmography signal can be indicative of an increase in the stress and/or pain level of the subject. Similarly, for example, an area (or portion) of the acquired photoplethysmography signal comprising an increase in the normalized amplitude of the acquired photoplethysmography signal can be indicative of a decrease in the stress and/or pain level of the subject. In some embodiments, a normalized amplitude of the acquired photoplethysmography signal that is above a predefined threshold may be indicative of the subject being in a relaxed state, whereas a normalized amplitude of the acquired photoplethysmography signal that is below the predefined threshold may be indicative of the subject experiencing a stressful and/or painful event. In general, a larger normalized amplitude of the acquired photoplethysmography signal can be indicative of the subject having a lower stress and/or pain level (e.g. the subject is in a more relaxed state), whereas a smaller normalized amplitude of the acquired photoplethysmography signal can be indicative of the subject having a higher stress and/or pain level. Thus, according to some embodiments, the smaller the normalized amplitude of the acquired photoplethysmography signal, the higher the stress and/or pain level of the subject.

In some embodiments, the stress and/or pain level of the subject may be determined from the acquired photoplethysmography signal by comparing the normalized characteristic of the acquired photoplethysmography signal to reference data stored in a memory (such as the memory mentioned earlier). The stored reference data may, for example, comprise training data. The training data can be data that is learnt from one or more other subjects (e.g. from a population) where stressful and/or painful stimuli are known and corresponding characteristics of the acquired photoplethysmography signal are recorded, such that the stress and/or pain response to the stressful and/or painful stimuli is also known. Thus, in some embodiments, the pain and/or stress level of the subject may be determined from the acquired photoplethysmography signal by comparing the normalized characteristic of the acquired photoplethysmography signal to reference characteristics for the photoplethysmography signal, which are stored with corresponding stress and/or pain levels. For example, in some embodiments, the pain and/or stress level of the subject may be determined as the stored pain and/or stress level that corresponds to the reference characteristic that most closely matches (e.g. differs the least from or is most similar to) the normalized characteristic of the acquired photoplethysmography signal.

Alternatively or in addition, in some embodiments, the stress and/or pain level of the subject may be determined from the acquired photoplethysmography signal by comparing the normalized characteristic of the acquired photoplethysmography signal to a characteristic of the acquired photoplethysmography signal for the subject in a well-defined reference phase of the subject. In this way, according to some embodiments, the normalized characteristic of the acquired photoplethysmography signal can be compared to a baseline, such that it can be identified when the normalized characteristic is reflecting that the subject is stressed and/or in pain, e.g. due to a stressful and/or painful stimulus. The degree by which the normalized characteristic differs from the baseline can be indicative of the stress and/or pain level of the subject according to these embodiments.

A well-defined reference phase of the subject can be any phase in which the subject is detected to be in a relaxed state, such as a phase in which the subject is detected to be asleep or in a certain sleep stage, a phase in which the subject is detected to be in a pre-surgical relaxed state (e.g. before a painful and/or stressful situation begins, such as before an initial incision, or after administration of anesthesia to the subject and before the painful procedure begins), a phase in which the subject is detected to be still, a phase in which the subject indicates (e.g. via a user interface 110 such as that mentioned earlier) that they are in a relaxed state, or any other well-defined reference phase of the subject. The subject may be detected to be in a relaxed state using, for example, one or more cameras (e.g. when an image acquired from one or more cameras shows the subject in a relaxed state) and/or one or more vital signs sensors (e.g. when one or more vital signs sensors detect a low heart rate or low blood pressure), where a wearable device may comprise one or more vital signs sensors according to some embodiments. As the characteristic of the acquired photoplethysmography signal identified at block 204 of Fig. 2 is normalized in the manner described earlier, the stress and/or pain level of the subject determined at block 206 of Fig. 2 is more accurate and reliable, since the effect of blood pressure (such as pulse pressure, mean blood pressure, etc.) is reduced or can at least be discriminated from a pain and/or stress response. As mentioned earlier, the characteristic of the acquired photoplethysmography signal can be normalized for blood pressure in a variety of ways, some of which will now be described.

In embodiments where the photoplethysmography sensor 104 is configured to obtain the photoplethysmography signal from a central part of the body of the subject, the processor 102 of the apparatus 100 can be configured to acquire, from the photoplethysmography sensor 104, the photoplethysmography signal obtained from the central part of the body of the subject. In these embodiments, for example, the characteristic of the acquired photoplethysmography signal can be normalized for blood pressure by the processor 102 of the apparatus 100 being configured to acquire the photoplethysmography signal obtained from the central part of the body of the subject. The acquired photoplethysmography signal can be normalized for blood pressure in this way since central parts of the body of the subject are influenced less by changes in blood pressure than peripheral parts of the body of the subject.

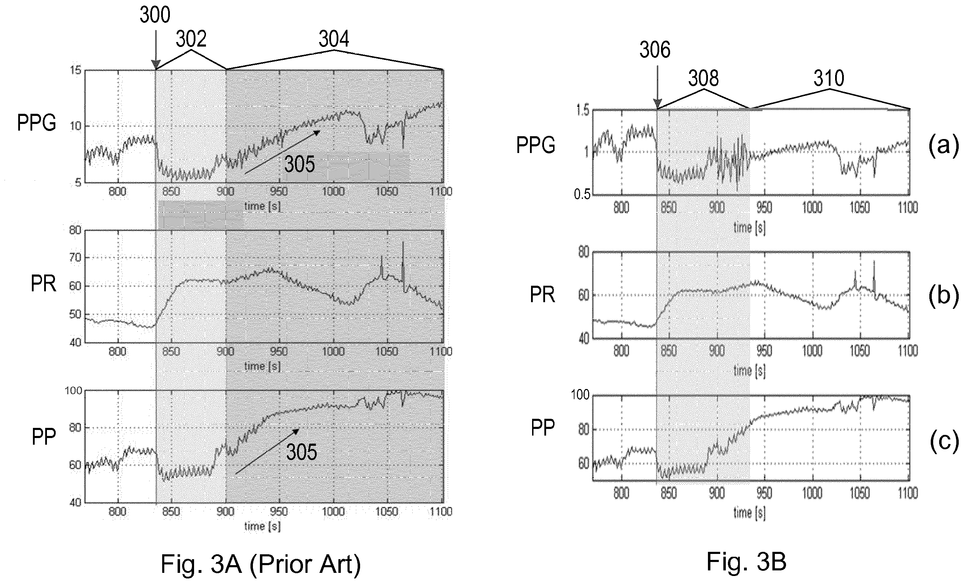

Fig. 3A is an illustration of signals acquired using an existing technique and Fig. 3B is an illustration of the same signals acquired using an apparatus according to such an embodiment. More specifically, Figs. 3A(a), (b) and (c) are illustrations of a photoplethysmography (PPG) signal, a pulse rate (PR), and a pulse pressure (PP) respectively, which are acquired using an existing technique. The existing technique is the technique that involves acquiring the signals from a peripheral part of the body (which, in this illustrated example, is a finger) of the subject.

Figs. 3B(a), (b) and (c) are illustrations of a photoplethysmography (PPG) signal, a pulse rate (PR), and a pulse pressure (PP) respectively, which are acquired using the apparatus 100 according to the embodiment described earlier, where the characteristic of the acquired photoplethysmography signal is normalized for blood pressure. In more detail, in the illustrated example embodiment of Fig. 3B, the characteristic of the acquired photoplethysmography signal is normalized for blood pressure by the processor 102 of the apparatus 100 acquiring the photoplethysmography signal obtained from the central part of the body of the subject. In the illustrated example of Fig. 3A and 3B, the characteristic of the acquired photoplethysmography signals on which the stress and/or pain level of the subject determination is based is the amplitude of the acquired photoplethysmography signals.

In the illustrated example of Fig. 3A and 3B, the signals are acquired during an abdominal surgery performed on the subject. The first incision made to the subject is indicated in Fig. 3A by the arrow 300 and in Fig. 3B by the arrow 306. The first incision is considered a painful and/or stressful event for the subject. This is illustrated, for example, by the strong increase in pulse rate at the start of the region labelled 302 in Fig. 3A(b) and at the start of the region labelled 308 in Fig. 3B(b). As illustrated within the shaded region labelled 302 in Fig. 3A(a) and the shaded region labelled 308 in Fig. 3B(a), during and immediately after the first incision, an expected decrease in amplitude of the measured photoplethysmography signals is observed. The decrease in amplitude is expected since, as a result of an incision, the autonomous nervous system triggers smooth muscle around vessels to constrict in order to centralize essential body functions. Based on this process, a larger amplitude of the measured photoplethysmography signal indicates a more relaxed patient, whereas a smaller amplitude of the measured photoplethysmography signal indicates that the subject is experiencing a stressful and/or painful event. Thus, with both the existing technique and the apparatus according to the mentioned embodiment, the amplitude of the measured photoplethysmography signals responds correctly in this region 302, 308 to show an initial pain and/or stress response of the subject due to the first incision.

However, as illustrated within the region labelled 304 in Fig. 3A(a), the strong pulse pressure (PP) response illustrated in Fig. 3A(c) later dominates the decrease in amplitude of the photoplethysmography signal acquired using the existing technique. As can be seen within the region labelled 304 in Fig. 3A(a), this results in an increase in amplitude of the photoplethysmography signal acquired using the existing technique, where the increase in amplitude is labelled by way of the arrow 305. According to the existing technique, the increase in amplitude labelled by way of the arrow 305 is clinically interpreted as the pain and/or stress level of the subject decreasing (i.e. the subject experiencing less pain and/or stress), which is incorrect. Thus, as illustrated in Fig. 3A(a), the amplitude of a photoplethysmography signal measured from a peripheral part of the body of the subject according to the existing technique is an unreliable and inaccurate indicator for painful and/or stressful events in relation to blood pressure changes.

In contrast, as illustrated within the regions labelled 308 and 310 of Fig. 3B (a), when the characteristic of the acquired photoplethysmography signal is normalized for blood pressure by the processor 102 of the apparatus 100 according to the mentioned embodiment acquiring the photoplethysmography signal from the central part of the body of the subject, the effect of the pulse pressure (PP) on the amplitude of the acquired photoplethysmography signal is reduced or even eliminated. As such, it can be seen from the region labelled 308 of Fig. 3B(a) that the subject actually suffers pain and/or stress over a longer period of time than that indicated by the region labelled 302 of Fig 3A(a). Thus, as illustrated in Fig. 3B(a), the amplitude of the photoplethysmography signal normalized for blood pressure according to the mentioned embodiment is a more reliable and more accurate indicator for painful and/or stressful events in relation to blood pressure changes.

Alternatively or in addition to the earlier described embodiment for the manner in which the characteristic can be normalized for blood pressure, in some embodiments, where the photoplethysmography sensor 104 is configured to obtain the photoplethysmography signal from a peripheral part of the body of the subject, the processor 102 of the apparatus 100 can be configured to acquire, from the photoplethysmography sensor 104, the photoplethysmography signal obtained from the peripheral part of the body of the subject. In these embodiments, the characteristic of the acquired photoplethysmography signal can be normalized for blood pressure by the processor 102 of the apparatus 100 being configured to normalize the characteristic with a blood pressure measurement for the subject to obtain the characteristic normalized for blood pressure.

The processor 102 of the apparatus 100 can be configured to acquire the blood pressure measurement for the subject from a blood pressure sensor 112. As mentioned earlier, the apparatus 100 may comprise the blood pressure sensor 112 or the blood pressure sensor 112 may be external to (i.e. separate to or remote from) the apparatus 100. The acquired blood pressure measurement for the subject that is used to normalize the characteristic of the acquired photoplethysmography signal can, for example, comprise any one or more of a systolic blood pressure (SBP) measurement, a diastolic blood pressure (DBP) measurement, a mean blood pressure (MBP) measurement, or a pulse pressure (PP) measurement. In some embodiments, the processor 102 can be configured to normalize the characteristic of the acquired photoplethysmography signal with the blood pressure measurement for the subject by determining a ratio of the characteristic of the acquired photoplethysmography signal to the blood pressure measurement for the subject to obtain the characteristic normalized for blood pressure.

The determined ratio $R$ of the characteristic of the acquired photoplethysmography signal to the blood pressure measurement for the subject according to this embodiment may be expressed as:

$$R = \frac{PPG}{BP}$$

where $PPG$ is the characteristic of the acquired photoplethysmography signal for the subject (e.g. PPGA or PPGA divided by a DC component of the acquired photoplethysmography signal) and $BP$ is the acquired or estimated blood pressure measurement (e.g. SBP, DBP, MBP, or PP) for the subject.

In some embodiments, the determined ratio of the characteristic of the acquired photoplethysmography signal to the blood pressure measurement for the subject may be compared to training data. For example, in some embodiments, the determined ratio of the characteristic of the acquired photoplethysmography signal to the blood pressure measurement for the subject may be compared to a reference ratio value determined from photoplethysmography signals and blood pressure measurements acquired from one or more other subjects (e.g. from a population). Alternatively or in addition, in some embodiments, the determined ratio of the characteristic of the acquired photoplethysmography signal to the blood pressure measurement for the subject may be compared to a ratio of the characteristic of the acquired photoplethysmography signal to the blood pressure measurement for the subject in a well-defined reference phase of the subject.

The well-defined reference phase of the subject may, for example, be at a time t0. According to some embodiments, the time t0 at which the subject is in a well-defined reference phase may be identified by detecting stimuli indicative of whether the subject is in such a phase. As mentioned earlier, a well-defined reference phase can be any phase in which the subject is detected to be in a relaxed state. In some embodiments, the comparison $R_N$ of the determined ratio of the characteristic of the acquired photoplethysmography signal to the blood pressure measurement for the subject to a ratio of the characteristic of the acquired photoplethysmography signal to the blood pressure measurement for the subject in a well-defined reference phase of the subject may be expressed as:

$$R_N = \frac{\dfrac{PPG(t)}{BP(t)}}{\dfrac{PPG(t_0)}{BP(t_0)}}$$

where $\dfrac{PPG(t)}{BP(t)}$ is the determined ratio of the characteristic of the acquired photoplethysmography signal for the subject to the acquired blood pressure measurement (e.g. SBP, DBP, MBP, or PP) for the subject and $\dfrac{PPG(t_0)}{BP(t_0)}$ is the ratio of the characteristic of the acquired photoplethysmography signal for the subject to the acquired blood pressure measurement (e.g. SBP, DBP, MBP, or PP) for the subject at a time $t_0$ in a well-defined reference phase of the subject.

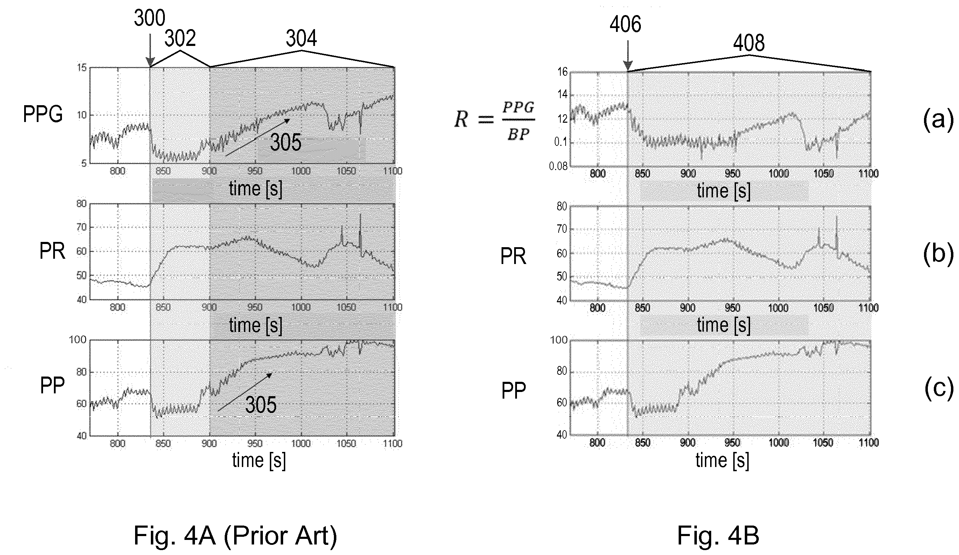

Fig. 4A is an illustration of signals acquired using an existing technique and Fig. 4B is an illustration of the same signals acquired using an apparatus according to such an embodiment. More specifically, Figs. 4A(a), (b) and (c) are illustrations of a photoplethysmography (PPG) signal, a pulse rate (PR), and a pulse pressure (PP) respectively, which are acquired using an existing technique. The existing technique is that described earlier with reference to Fig. 3A. Thus, the description of Fig. 3A will not be repeated here but will be understood to also apply to Fig. 4A.

Figs. 4B(a), (b) and (c) are illustrations of a photoplethysmography (PPG) signal, a pulse rate (PR), and a pulse pressure (PP) respectively, which are acquired using the apparatus 100 according to the embodiment described earlier, where the characteristic of the acquired photoplethysmography signal is normalized for blood pressure. In more detail, in the illustrated example embodiment of Fig. 4B, the characteristic of the acquired photoplethysmography signal is normalized for blood pressure by the processor 102 of the apparatus 100 determining a ratio R of a characteristic of the acquired photoplethysmography signal to a blood pressure measurement for the subject. In the illustrated example of Fig. 4A and 4B, the characteristic of the acquired photoplethysmography signals on which the stress and/or pain level of the subject determination is based is the amplitude of the acquired photoplethysmography signals. In Fig. 4B, the first incision made to the subject is indicated by the arrow 406. The first incision is considered a stressful and/or event painful for the subject. This is illustrated, for example, by the strong increase in pulse rate at the start of the region labelled 302 in Fig. 4A(b) and at the start of the region labelled 408 in Fig. 4B(b).

In contrast to the existing technique of Fig. 4A, as illustrated within the region labelled 408 of Fig. 4B(a), when the characteristic of the acquired photoplethysmography signal is normalized for blood pressure by the processor 102 of the apparatus 100 according to the mentioned embodiment determining a ratio of a characteristic of the acquired photoplethysmography signal to a blood pressure measurement for the subject, the amplitude of the acquired photoplethysmography signal remains low for a longer period of time. In Fig. 4B(a), the acquired photoplethysmography signal is not as strongly correlated with the pulse pressure (PP) as it is in Fig. 4A(a). In fact, it can be seen from the region labelled 408 in Fig. 4B that the amplitude of the acquired photoplethysmography signal shown in Fig. 4B(a) remains low even when the pulse pressure (PP) shown in Fig. 4B(c) starts to increase. This indicates that the reason for the amplitude of the acquired photoplethysmography signal remaining low in Fig. 4B(a) is not only due to a low pulse pressure (PP), as may be determined to be the case in Fig 4A, but also due to the subject suffering a prolonged state of pain and/or stress. Thus, as illustrated in Fig. 4B(a), the amplitude of the photoplethysmography signal normalized for blood pressure according to the mentioned embodiment is a more reliable and more accurate indicator for painful and/or stressful events in relation to blood pressure changes.

Alternatively or in addition to the earlier described embodiments for the manner in which the characteristic can be normalized for blood pressure, in some embodiments, the processor 102 can be configured to acquire, from a first photoplethysmography sensor 104, a first photoplethysmography signal obtained from a first part of the body of the subject and can be further configured to acquire, from a second photoplethysmography sensor 104, a second photoplethysmography signal obtained from a second part of the body of the subject. The second photoplethysmography sensor 104 may be the same photoplethysmography sensor as the first photoplethysmography sensor 104 according to some embodiments or a different photoplethysmography sensor as the first photoplethysmography sensor 104 according to other embodiments. In some embodiments, the first part of the body of the subject may be different to the second part of the body of the subject. For example, according to some embodiments, one of the first part of the body of the subject and the second part of the body of the subject can be a peripheral part (e.g. a finger or any other peripheral part) of the body of the subject, and the other of the first part of the body of the subject and the second part of the body of the subject can be a central part (e.g. a forehead or any other central part) of the body of the subject.

In embodiments where the processor 102 is configured to acquire a first photoplethysmography signal and a second photoplethysmography signal, the processor 102 can be configured to identify a first characteristic of the first photoplethysmography signal and a corresponding second characteristic of the second photoplethysmography signal. A second characteristic is "corresponding" where the second characteristic is the same (or the same type of) characteristic as the first characteristic. For example, where the first characteristic comprises an amplitude of the acquired first photoplethysmography signal, a corresponding second characteristic comprises an amplitude of the acquired second photoplethysmography signal. In these embodiments, the characteristic can be normalized for blood pressure by the processor 102 being configured to normalize the first characteristic with the corresponding second characteristic to obtain the characteristic normalized for blood pressure. For example, in some embodiments, the processor 102 can be configured to normalize the first characteristic with the second characteristic by determining a ratio of the first characteristic to the second characteristic to obtain the characteristic normalized for blood pressure.

The characteristic normalized for blood pressure H according to this embodiment may be expressed as:

$$ H = \frac{PPG_1}{PPG_2} $$

where $PPG_1$ is the first characteristic of the first photoplethysmography signal (e.g. obtained from a peripheral part of the body of the subject) and $PPG_2$ is the second characteristic of a second photoplethysmography signal (e.g. obtained from a central part of the body of the subject).

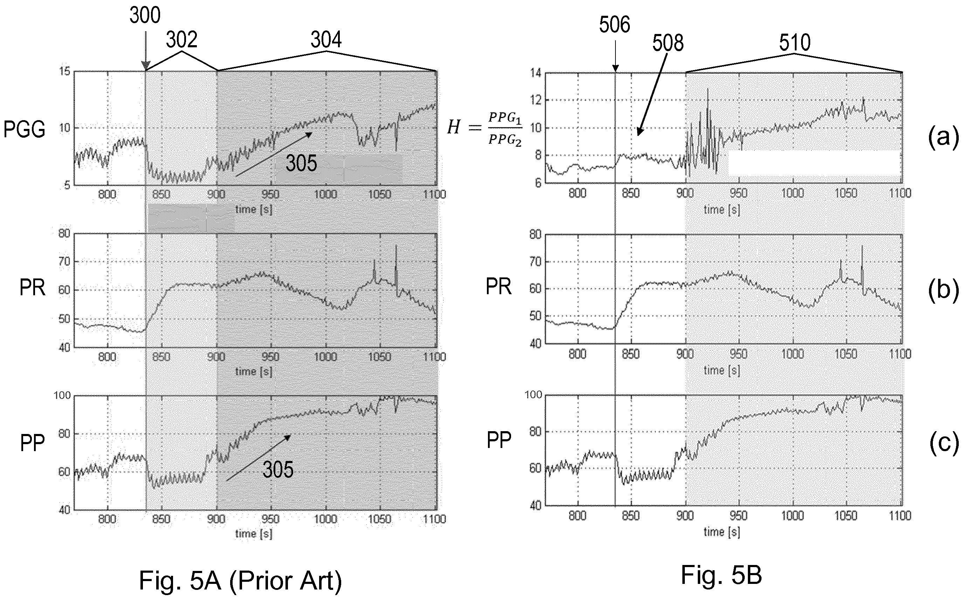

Fig. 5A is an illustration of signals acquired using an existing technique and Fig. 5B is an illustration of the same signals acquired using an apparatus according to such an embodiment. More specifically, Figs. 5A(a), (b) and (c) are illustrations of a photoplethysmography (PPG) signal, a pulse rate (PR), and a pulse pressure (PP) respectively, which are acquired using an existing technique. The existing technique is that described earlier with reference to Fig. 3A. Thus, the description of Fig. 3A will not be repeated here but will be understood to also apply to Fig. 5A.

Figs. 5B (a), (b) and (c) are illustrations of a photoplethysmography (PPG) signal, a pulse rate (PR), and a pulse pressure (PP) respectively, which are acquired using the apparatus 100 according to the embodiment described earlier, where the characteristic of the acquired photoplethysmography signal is normalized for blood pressure. In more detail, in the illustrated example embodiment of Fig. 5B, the characteristic of the acquired photoplethysmography signal is normalized for blood pressure by the processor 102 of the apparatus 100 determining a ratio H of a first characteristic of a first photoplethysmography signal obtained from a peripheral part of the body (e.g. a finger) of the subject to a second characteristic of a second photoplethysmography signal obtained from a central part of the body (e.g. a forehead) of the subject. In the illustrated example of Fig. 5A and 5B, the characteristic of the acquired photoplethysmography signal on which the stress and/or pain level of the subject determination is based is the amplitude of the acquired photoplethysmography signal. In Fig. 5B, the first incision made to the subject is indicated by the arrow 506. The first incision is considered a stressful and/or painful event for the subject. This is illustrated, for example, by the strong increase in pulse rate at the start of the region labelled 302 in Fig. 5A(b) and at the start of the region labelled 508 in Fig. 4B(b).

In contrast to the existing technique of Fig. 5A, as illustrated within the region labelled 508 of Fig. 5B (a), the initial pain and/or stress response of the subject due to the first incision is suppressed when the characteristic of the acquired photoplethysmography signal is normalized for blood pressure by the processor 102 of the apparatus 100 according to the mentioned embodiment by determining a ratio of a first characteristic of a first photoplethysmography signal obtained from a peripheral part of the body (e.g. a finger) of the subject to a second characteristic of a second photoplethysmography signal obtained from a central part of the body (e.g. a forehead) of the subject. However, the consecutive increase in the pulse pressure in the region labelled 510 of Fig. 5B(c) is still clearly visible in the amplitude of the acquired photoplethysmography signal illustrated in the region labelled 510 of Fig. 5B(a). As such, it can be seen from the region labelled 508 of Fig. 5B(a) that the subject actually suffers from a blood pressure increase, rather than an increase in the level of pain and/or stress. Thus, as illustrated in Fig. 5B(a), the amplitude of the photoplethysmography signal normalized for blood pressure according to the mentioned embodiment is a more reliable and more specific indicator for painful and/or stressful events, since these events can be discriminated from blood pressure changes.

Although it has been described that photoplethysmography signals obtained from first and second parts of the body of the subject can be acquired, in some embodiments, the processor 102 can also be configured to acquire a photoplethysmography signal obtained from at least one other part of the body of the subject. In these embodiments, a corresponding characteristic of the photoplethysmography signal obtained from the at least one other part of the body of the subject may also be identified and used in normalizing the first characteristic together with the corresponding second characteristic to obtain the characteristic normalized for blood pressure.

Alternatively or in addition to the earlier described embodiments for the manner in which the characteristic can be normalized for blood pressure, in some embodiments, the processor 102 can be configured to normalize the characteristic for blood pressure by determining a pulse transit time (PTT) for the subject and normalizing the characteristic of the acquired photoplethysmography signal with the determined pulse transit time for the subject to obtain the characteristic normalized for blood pressure. Herein, the pulse transit time for the subject can be defined as the time it takes for a pulse pressure wave to propagate from one arterial site of the body of the subject to another arterial site of the body of the subject. An arterial site of the body of the subject can be any site of the body of the subject at which a pulse pressure wave can be detected (e.g. any site of the body of the subject comprising a pulsatile blood vessel (e.g. artery, arteriole, or any other pulsatile blood vessel)). In some embodiments, the processor 102 can be configured to normalize the characteristic with the determined pulse transit time by determining a ratio of the characteristic to the determined pulse transit time for the subject to obtain the characteristic normalized for blood pressure.

The determined ratio $R'$ of the characteristic to the determined pulse transit time for the subject according to this embodiment may be expressed as:

$$R' = \frac{PPG}{PTT}$$

where $PPG$ is the characteristic of the acquired photoplethysmography signal for the subject (e.g. PPGA or PPGA divided by a DC component of the acquired photoplethysmography signal) and $PTT$ is the pulse transit time for the subject.

In some embodiments, the pulse transit time for the subject can be determined using an electrocardiogram signal obtained from the subject and the acquired photoplethysmography signal for the subject. The pulse transit time for the subject has an inverse relationship to systolic arterial blood pressure and can thus be used as an indirect surrogate to estimate blood pressure of the subject. In some embodiments, the pulse transit time for the subject may be used to normalize the characteristic when the blood pressure (e.g. neither cuff nor arterial blood pressure) of the subject is unavailable. In some embodiments, the pulse transit time for the subject may be determined by measuring a time delay between any characteristic point (e.g. the R-peak, or any other characteristic point) of an electrocardiogram signal obtained from the subject and any characteristic point (e.g. a first derivative of the maximum point, a second derivative of the maximum point, an intersecting tangent point, or any other characteristic point) of the acquired photoplethysmography signal). Although a variety of ways in which the characteristic of the acquired photoplethysmography signal can be normalized for blood pressure have been described alone, it will be understood that any of the described ways in which the characteristic of the acquired photoplethysmography signal can be normalized for blood pressure may equally be used in combination with any other. For example, in some embodiments, the characteristic of the acquired photoplethysmography signal may be normalized for blood pressure using more than one of the ways described herein.

In some embodiments, the acquired photoplethysmography signal with the characteristic normalized for blood pressure may be rendered (e.g. output, displayed, or provided) by a user interface 110. For example, in some embodiments, the processor 102 of the apparatus 100 may control the user interface 110 to render (e.g. output, display, or provide) the acquired photoplethysmography signal with the characteristic normalized for blood pressure. As mentioned earlier, the apparatus 100 may comprise the user interface 110 or the user interface 110 may be external to (i.e. separate to or remote from) the apparatus 100. In some embodiments, the acquired photoplethysmography signal with the characteristic normalized for blood pressure may be rendered with other information, such as any one or more of indication of areas of reliable characteristics (e.g. areas of characteristics that are due to pain and/or stress as opposed to an increase in blood pressure), a pulse rate (PR) of the subject, and a pressure pulse (PP) of the subject. In this way, for example, it is possible to provide improved clinical decision support to medical professional (e.g. a doctor, an anesthetist, or similar). In embodiments where the characteristic of the acquired photoplethysmography signal is normalized for blood pressure using more than one of the ways described herein, a plurality of photoplethysmography signal can be provided with the characteristic normalized for blood pressure in different ways. Thus, in some of these embodiments, two or more photoplethysmography signals with the characteristic normalized for blood pressure in different ways may be rendered by the user interface 110, e.g. simultaneously.

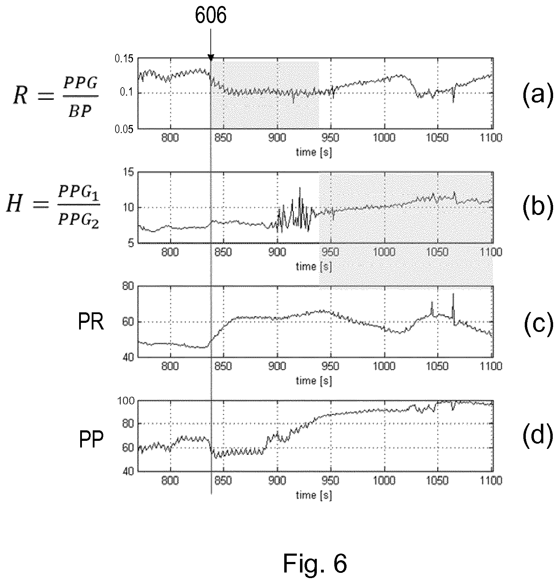

Fig. 6 is an illustration of signals acquired using an apparatus according to such an embodiment. More specifically, Fig. 6 is an illustration of two photoplethysmography signals with the characteristic normalized for blood pressure in two different ways. In Fig. 6, a first incision made to the subject is indicated by the arrow 606. Fig. 6(a) is an illustration of a photoplethysmography signal with the characteristic normalized for blood pressure by determining a ratio R of the characteristic of the acquired photoplethysmography signal to the blood pressure measurement for the subject. Fig. 6(b) is an illustration of a photoplethysmography signal with the characteristic normalized for blood pressure by determining a ratio H of a first characteristic of a first photoplethysmography signal obtained from a peripheral part of the body of the subject to a second characteristic of a second photoplethysmography signal obtained from a central part of the of the subject.

As illustrated in Fig. 6, in some embodiments, the two photoplethysmography signals with the characteristic normalized for blood pressure in two different ways may be rendered by the user interface 110 simultaneously. In some embodiments, as illustrated in Fig. 6(c) and 6(d) respectively, the pulse rate (PR) of the subject and the pressure pulse (PP) of the subject also may be rendered by the user interface 110 simultaneously with the photoplethysmography signals. In this way, a pain and/or stress response and a blood pressure related effect can be discriminated from one another. For example, the pain and/or stress response and the blood pressure related effect can be discriminated from one another during the first incision 606. This can, for example, provide a user of the user interface 110 (e.g. a medical professional, such as a doctor, an anesthetist, etc.) with better guidance when operating on the subject.

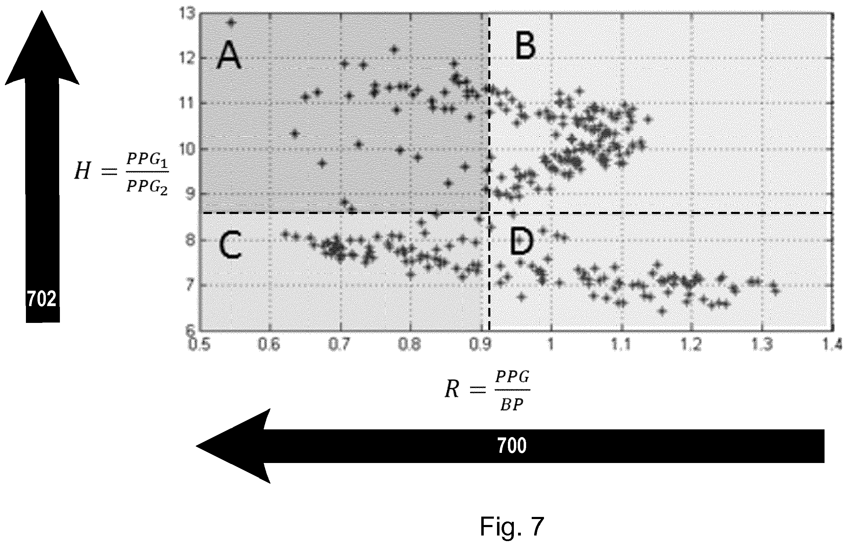

Fig. 7 is an illustration of an example output of signals acquired using an apparatus according to some embodiments described herein. More specifically, Fig. 7 illustrates another example manner in which two photoplethysmography signals with the characteristic normalized for blood pressure in two different ways may be rendered by the user interface 110. The horizontal axis of Fig. 7 represents data of a photoplethysmography signal with the characteristic normalized for blood pressure by determining a ratio R of the characteristic of the acquired photoplethysmography signal to the blood pressure measurement for the subject or by acquiring the photoplethysmography signal obtained from the central part of the body of the subject. The vertical axis of Fig. 7 represents data of the photoplethysmography signal with the characteristic normalized for blood pressure by determining a ratio H of a first characteristic of a first photoplethysmography signal obtained from a peripheral part of the body of the subject to a second characteristic of a second photoplethysmography signal obtained from a central part of the of the subject. Thus, in some embodiments, a photoplethysmography signal may be rendered by plotting data of the photoplethysmography signal with the characteristic normalized for blood pressure in one way may against data of the photoplethysmography signal with the characteristic normalized for blood pressure in another way. By visualizing the photoplethysmography signal in this way, a user of the user interface 110 (e.g. a medical professional, such as a doctor, an anesthetist, etc.) can be provided with an improved assessment of the subject in terms of a pain and/or stress response of the subject.

In some embodiments, it may be possible to discriminate a pain and/or stress response of the subject from a blood pressure event. For example, in the illustrated embodiment of Fig. 7, the arrow labelled 700 is indicative of increasing pain and/or stress experienced by the subject and the arrow labelled 702 is indicative of an increasing probability of a hemodynamic (blood pressure) event occurring. Thus, in the example embodiment illustrated in Fig. 7, the area labelled A can be interpreted as a hemodynamic (blood pressure) event and the subject experiencing pain and/or stress, the area labelled B can be interpreted as a hemodynamic (blood pressure) event, the area labelled C can be interpreted as the subject experiencing pain and/or stress, and the area labelled D can be interpreted as the subject being in a relaxed state (which may be used as a reference).

In addition to the apparatus 100 and method 200 described earlier, there is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved apparatus, method and computer program product for determining a stress and/or pain level of a subject.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. An apparatus (100) for determining a stress and/or pain level of a subject, the apparatus (100) comprising a processor (102) configured to:

   acquire, from a photoplethysmography sensor (104), a photoplethysmography signal obtained from the subject;
   identify a characteristic of the acquired photoplethysmography signal, wherein the characteristic is normalized for blood pressure; and determine the stress and/or pain level of the subject from the acquired photoplethysmography signal based on the normalized characteristic.

2. The apparatus (100) as claimed in claim 1, wherein the characteristic is normalized for blood pressure by the processor (102) being configured to:
acquire, from the photoplethysmography sensor (104), a photoplethysmography signal obtained from a central part of the body of the subject.

3. The apparatus (100) as claimed in any one of claims 1 or 2, wherein the characteristic is normalized for blood pressure by the processor (102) being configured to:

   acquire, from the photoplethysmography sensor (104), a photoplethysmography signal obtained from a peripheral part of the body of the subject;
   acquire, from a blood pressure sensor (112), a blood pressure measurement for the subject; and
   normalize the characteristic with the blood pressure measurement for the subject to obtain the characteristic normalized for blood pressure.

4. The apparatus (100) as claimed in claim 3, wherein the processor (102) is configured to normalize the characteristic with the blood pressure measurement for the subject by:
determining a ratio of the characteristic to the blood pressure measurement for the subject to obtain the characteristic normalized for blood pressure.

5. The apparatus (100) as claimed in any one of claims 3 or 4, wherein the blood pressure measurement comprises a systolic blood pressure measurement, a diastolic blood pressure measurement, a mean blood pressure measurement, or a pulse pressure measurement.

6. The apparatus (100) as claimed in any one of the preceding claims, wherein the characteristic is normalized for blood pressure by the processor (102) being configured to:

   acquire, from a first photoplethysmography sensor (104), a first photoplethysmography signal obtained from a first part of the body of the subject;
   acquire, from a second photoplethysmography sensor (104), a second photoplethysmography signal obtained from a second part of the body of the subject;
   identify a first characteristic of the first photoplethysmography signal and a corresponding second characteristic of the second photoplethysmography signal; and
normalize the first characteristic with the corresponding second characteristic to obtain the characteristic normalized for blood pressure.

7. The apparatus (100) as claimed in claim 6, wherein the first part of the body of the subject is different to the second part of the body of the subject.

8. The apparatus (100) as claimed in any of claims 6 or 7, wherein:

   one of the first part of the body of the subject and the second part of the body of the subject is a peripheral part of the body of the subject; and the other of the first part of the body of the subject and the second part of the body of the subject is a central part of the body of the subject.

9. The apparatus (100) as claimed in any one of claims 6, 7 or 8, wherein the processor (102) is configured to normalize the first characteristic with the second characteristic by:
determining a ratio of the first characteristic to the second characteristic to obtain the characteristic normalized for blood pressure.

10. The apparatus (100) as claimed in any of the preceding claims, wherein the processor (102) is configured to normalize the characteristic for blood pressure by:

   determining a pulse transit time for the subject; and
   normalizing the characteristic with the determined pulse transit time for the subject to obtain the characteristic normalized for blood pressure.

11. The apparatus (100) as claimed in claim 10, wherein the processor (102) is configured to normalize the characteristic with the determined pulse transit time for the subject by:
determining a ratio of the characteristic to the determined pulse transit time for the subject to obtain the characteristic normalized for blood pressure.

12. The apparatus (100) as claimed in any one of the preceding claims, wherein the processor (102) is configured to determine the stress and/or pain level of the subject by:
identifying whether one or more areas of the acquired photoplethysmography signal are indicative of an increase in the stress and/or pain level of the subject based on the normalized characteristic.

13. The apparatus (100) as claimed in any one of the preceding claims, wherein the characteristic of the acquired photoplethysmography signal comprises an amplitude of the acquired photoplethysmography signal.

14. A method (200) for determining a stress and/or pain level of a subject, the method (200) comprising:

    acquiring (202), from a photoplethysmography sensor (104), a photoplethysmography signal obtained from the subject;
    identifying (204) a characteristic of the acquired photoplethysmography signal, wherein the characteristic is normalized for blood pressure; and
    determining (206) the stress and/or pain level of the subject from the acquired photoplethysmography signal based on the normalized characteristic.

15. A computer program product comprising a computer readable medium, the computer readable medium having computer readable code embodied therein, the computer readable code being configured such that, on execution by a suitable computer or processor, the computer or processor is caused to perform the method as claimed in claim 14.

Fig. 1

Fig. 2

Fig. 3B

Fig. 3A (Prior Art)

(a)

(b)

(c)

408

406

time [s]

$R = \dfrac{PPG}{BP}$

PR

PP

Fig. 4B

300

302

304

305

305

time [s]

PPG

PR

PP

Fig. 4A (Prior Art)

$$H = \frac{PPG_1}{PPG_2}$$

Fig. 5A (Prior Art)     Fig. 5B

EP 3 563 759 A1

$$R = \frac{PPG}{BP}$$

(a)

$$H = \frac{PPG_1}{PPG_2}$$

(b)

PR

(c)

PP

(d)

Fig. 6

Fig. 7

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/242955 A1 (UUTELA KIMMO [FI] ET AL) 2 October 2008 (2008-10-02) * paragraphs [0012], [0016], [0033], [0041], [0043], [0044], [0047] - [0053], [0066] - [0070] * | 1-15 | INV. A61B5/021 A61B5/0295 A61B5/00 ADD. A61B5/024 |
| X | GOHICHI TANAKA ET AL: "Examination of normalized pulse volume-blood volume relationship: toward a more valid estimation of the finger sympathetic tone", INTERNATIONAL JOURNAL OF PSYCHOPHYSIOLOGY, vol. 48, no. 3, 1 June 2003 (2003-06-01), pages 293-306, XP055267410, NL ISSN: 0167-8760, DOI: 10.1016/S0167-8760(03)00056-4 * the whole document * * in particular: * * abstract * * sections 1, 2.3, 2.5, 4; * | 1-15 | |
| X | US 2010/249555 A1 (SETHI RAKESH [CA] ET AL) 30 September 2010 (2010-09-30) * paragraphs [0025], [0030], [0031], [0041], [0090] - [0093], [0100] - [0101] * | 1-15 | TECHNICAL FIELDS SEARCHED (IPC) A61B |

The present search report has been drawn up for all claims

| Place of search | Date of completion of the search | Examiner |
|---|---|---|
| The Hague | 17 October 2018 | Albrecht, Ronald |

EPO FORM 1503 03.82 (P04C01)

## ANNEX TO THE EUROPEAN SEARCH REPORT
## ON EUROPEAN PATENT APPLICATION NO.

EP 18 17 0248

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2018

| Patent document cited in search report | | Publication date | Patent family member(s) | | Publication date |
|---|---|---|---|---|---|
| US 2008242955 | A1 | 02-10-2008 | DE 102008016298 A1 | | 02-10-2008 |
| | | | NL 2001399 C2 | | 22-09-2009 |
| | | | US 2008242955 A1 | | 02-10-2008 |
| US 2010249555 | A1 | 30-09-2010 | NONE | | |

EPO FORM P0459

For more details about this annex : see Official Journal of the European Patent Office, No. 12/82